# EUROPEAN PATENT SPECIFICATION

(11) **EP 4 523 600 B1**
(45) Date of publication and mention of the grant of the patent: **12.08.2026**
(21) Application number: 23197400.7
(22) Date of filing: 14.09.2023
(51) Int. Cl.: A61B 1/00, A61B 1/05

(54) **MEDICAL VISUALIZATION SYSTEM**
MEDIZINISCHES VISUALISIERUNGSSYSTEM
SYSTÈME DE VISUALISATION MÉDICALE

(43) Date of publication of application: 19.03.2025
(60) Divisional application: 26190427.0
(73) Proprietor: Ambu A/S, 2750 Ballerup (DK)
(72) Inventor: MATTHISON-HANSEN, Kaspar Mat, 3000 Helsingør (DK)
(74) Representative: COPA Copenhagen Patents

(56) References cited:
- WO-A1-2020/006517
- WO-A1-2021/090056
- WO-A1-2021/242736
- US-A1- 2017 215 695

## Description

The present disclosure relates to a medical visualization system and elements thereof. Particularly, a medical visualization system comprising a medical visualization device, such as an endoscope, and a video processing apparatus.

### BACKGROUND

An endoscope may be utilized to visually examine certain areas of the body of a person, such as inside a body cavity of the person. For example, an endoscope may be used to inspect the airways, the digestive tract, or the intestines.

An endoscope may be provided with a camera and be attached to a video processing apparatus, such as a video processing apparatus with a display screen, a video output from the camera of the endoscope may be received and displayed at the video processing apparatus or a coupled display screen, thereby allowing an operator to control the endoscope to inspect an area of interest.

An endoscope may comprise an operating handle at a proximal end and an insertion cord extending from the handle towards a distal end. The handle is configured to be held by an operator and inter alia comprises externally protruding operating members connected to internal control means allowing the operator to control the movement of a bending section near the distal end of the insertion cord, while advancing the distal end of the insertion cord to a desired location, e.g. within a body cavity of a person. By means of a coupled video processing apparatus, such as a video processing apparatus with a display screen, the location to which the distal end has been advanced may be inspected using the endoscope. As an exemplary alternative to the endoscope comprising an insertion cord, the endoscope may be a laryngoscope, which is a more rigid type of endoscope, usually having an L-shape, used to inspect the larynx.

An endoscope may be a disposable endoscope. It is generally advantageous that endoscopes are disposable so as to avoid demanding cleaning processes and risks of cross contamination between patients. However, it follows logically from the concept of disposable devices that if devices are discarded after use it involves some waste of materials. Thus, reducing the amount of waste may reduce costs as well as environmental impact. The challenge is to find solutions reducing the amount of waste without compromising patient safety and medical personnels' ability to perform procedures efficiently and satisfactorily.

Medical visualization systems comprising a disposable part and a reusable part are known from prior art documents WO 2020/006517 A1, WO 2021/090056 A1 and WO 2021/242736 A1.

### SUMMARY

It is an object of the present disclosure to provide a solution which at least improves the solutions of the prior art. Particularly, it is an object of the present disclosure to provide a medical visualization system and elements thereof.

Particularly, a transmission component and an endoscope are disclosed as well as a medical visualization system comprising such a transmission component and endoscope.

The disclosed medical visualization system further comprises a video processing apparatus.

The endoscope comprises a camera module adapted to generate image data indicative of a view from the endoscope, a handle, and a device coupling part.

The video processing apparatus comprises a housing and a processor. The processor is adapted to receive the image data and/or data representative of the image data and adapted to cause a display to display a live representation of the image data.

The transmission component is adapted to transmit the image data and/or the data representative of the image data to the video processing apparatus. The transmission component comprises a component coupling part adapted to couple with the device coupling part of the endoscope.

Also disclosed is a cable reel and a support bracket for a medical visualization system, such as the disclosed medical visualization system. The cable reel and/or the support bracket may separately or collectively also form part of the disclosed visualization system.

Advantages of the present disclosure include a reduction of waste, e.g. when using disposable devices, such as disposable endoscopes, thereby reducing environmental impact as well as reducing costs. However, these advantages are also applicable for reusable devices. Particularly, since also reusable devices need replacement, and since the present disclosure provides that elements may be replaced individually. Furthermore, the present disclosure provides advantageous procedures for assembling various parts of the system, in particular in ways reducing physical contact with reusable elements. Further advantages of the present disclosure will become clear for the skilled person when reading the present description.

### GENERAL DESCRIPTION

The transmission component comprises a component processor. The component processor is adapted to receive the image data from the camera module. The component processor is further adapted to preprocess the image data to provide the data representative of the image data, which may be received by the video processing apparatus, such as the processor of the video processing apparatus. Thus, the transmission component transmits the data representative of the image data to the video processing apparatus, such as to the processor of the video processing apparatus.

The component coupling part comprises the component processor. Providing the component processor as part of the component coupling part may be advantageous so as to reduce the distance between the component processor and the camera module of the endoscope. Thereby, the signal between the camera module and the component processor travels a short distance, e.g. in comparison with the component processor being arranged in an opposite end of the transmission component or in the video processing apparatus. A short distance between the camera module and the component processor is beneficial to avoid and/or reduce noise in the signal.

The transmission component may transmit data and/or signals from the video processing apparatus to the endoscope, such as to the camera module and/or to the light emitter of the endoscope. For example, camera configuration data and/or light configuration data may be transmitted from the video processing apparatus to the endoscope/camera module/light emitter.

The component coupling part comprises one or more physical activatable input mechanisms.

The one or more physical activatable input mechanism may be or include one or more buttons, one or more levers, one or more switches or other suitable components known in the art. Activation of the one or more physical activatable input mechanisms causes the transmission component to transmit one or more input mechanism signals to the video processing apparatus indicative of activation of the one or more physical activatable input mechanisms. Such input mechanism signals may activate various functions of the video processing apparatus, e.g. capturing still images, starting/stopping video recording, changing predefined settings, etc.

The handle of the endoscope comprises one or more communicator elements, e.g. plungers, levers etc. The one or more communicator elements are adapted to activate the one or more physical activatable input mechanisms upon being manipulated by a user. Accordingly, a user may activate the one or more physical activatable input mechanisms (and activate a corresponding function of the video processing apparatus) by manipulating a respective communicator element. Thereby, the parts being touched by the user may be simple, non-electronic elements, which may be provided as part of a disposable element, e.g. the endoscope. In this way, the risk of cross contamination may be reduced in a cost-efficient manner.

For example, a first communicator element of the one or more communicator elements may extend from a first side to a second side. The first side may be arranged exteriorly of the handle. The second side may be arranged interiorly of the handle. The second side may be adapted to contact a first physical activatable input mechanism of the one or more physical activatable input mechanism when the component coupling part is coupled with the device coupling part. A second communicator element of the one or more communicator elements may extend from a first side to a second side. The second side of the second communicator element may be adapted to contact a second physical activatable input mechanism of the one or more physical activatable input mechanism when the component coupling part is coupled with the device coupling part.

The transmission component may comprise a device wireless transceiver. The video processing apparatus may comprise an apparatus wireless transceiver. The device wireless transceiver may be a transmitter and/or a receiver. The apparatus wireless transceiver may be a receiver and/or a transmitter. The device wireless transceiver may be adapted to wirelessly communicate with the apparatus wireless transceiver. The apparatus wireless transceiver may be adapted to wirelessly communicate with the device wireless transceiver. The device wireless transceiver may be adapted to wirelessly transmit the image data and/or the data representative of the image data to the apparatus wireless transceiver. The apparatus wireless transceiver may be adapted to wirelessly receive the image data and/or the data representative of the image data from the device wireless transceiver. The apparatus wireless transceiver may be adapted to wirelessly transmit data and/or signals, e.g. camera configuration data and/or light configuration data, to the device wireless transceiver. The device wireless transceiver may be adapted to wirelessly receive data and/or signals from the apparatus wireless transceiver.

The transmission component may comprise a device cable. The device cable may be provided alternative to the device wireless transceiver or in addition to the device wireless transceiver. The device cable may extend from a first cable end to a second cable end. The component coupling part may be provided at the second cable end. The first cable end may be connectable to the video processing apparatus. A connector, e.g. connectable to the video processing apparatus, may be provided at the first cable end.

The device cable may comprise a stiffened portion. The stiffened portion may extend from the second cable end and/or from the component coupling part towards the first cable end. The stiffened portion may have a length of between 20-150 mm from the second cable end, such as between 40-100 mm, such as between 60-80 mm.

The stiffened portion may be straight or substantially straight. Alternatively, the stiffened portion may be curved, e.g. upwards and/or away from the operator.

The handle may be an elongate element extending along a handle axis. The stiffened portion of the device cable may extend in a normal plane normal to the handle axis. The stiffened portion may extend at an angle relative to the normal plane and/or the stiffened portion may be curved upwards or downwards relative to the normal plane. The stiffened portion may extend between an upper plane and a lower plane. The upper plane may form an angle of less than +15 degrees relative to the normal plane. The lower plane may form an angle of less than -15 degrees relative to the normal plane. The lower plane may form an angle of 30 degrees relative to the upper plane. The lower plane, the upper plane and the normal plane may intersect the handle axis at a common point.

The handle may have a front side and a back side. The front side may be opposite the back side. The front side may be facing in a forward direction. The back side may be facing in a backward direction. The forward direction may be opposite the backward direction. The handle may be adapted to be gripped by a first hand of an operator, e.g. such that the back side of the handle is facing in the backwards direction towards the wrist of the first hand, and/or such that the front side is facing in the forward direction opposite the backward direction, i.e. away from the wrist of the first hand. The stiffened portion may extend in a cable direction. The cable direction may be between the forward direction and the backwards direction. For example, an angle between the cable direction and the forward direction may be between 5 and 85 degrees, such as between 40 degrees and 65 degrees, e.g. 60 degrees or 45 degrees.

As mentioned above, the present disclosure also comprises a cable reel for a medical visualization system, such as the disclosed medical visualization system. Accordingly, the medical visualization system may comprise the cable reel. The cable reel may be adapted to reel in and/or unreel at least a portion of the device cable.

The cable reel may be driven by an actuator, such as a rotary actuator, e.g. an electrical motor, e.g. a DC motor. The actuator may drive the cable reel such that the portion of the device cable is reeled in and out as appropriate. Such actuated drive of the cable reel may be advantageous for reducing influence of the cable reel on the operator's ability to control the endoscope. The actuator may be activated to reel in the portion of the device cable based on a first control input. The actuator may be activated to unreel the portion of the device cable based on a second control input. The first control input and/or the second control input may, for example, be caused by a push button, a switch, or similar. Alternatively or additionally, the first control input and/or the second control input may be generated by the video processing apparatus, e.g. based on a user input at the video processing apparatus. Alternatively or additionally, the first control input and/or the second control input may be generated in response to the component coupling part being received in and/or removed from a coupling part retainer.

The cable reel may comprise a cable reel housing. The cable reel housing may enclose the portion of the device cable when reeled in. The cable reel housing may comprise an opening. The device cable may extend through the opening. The opening may be covered with bristles or similar. The cable reel housing may be opaque, such as opaque to UV light.

The cable reel may comprise a spool. The spool may be accommodated in the cable reel housing. The portion of the device cable may be rolled onto the spool when reeled in by the cable reel. The spool may be transparent, such as transparent to UV light. For example, the spool may be made from Polymethylmethacrylate (PMMA).

The cable reel may comprise a UV light emitter and/or a plurality of UV light emitters. The UV light emitter(s) may be provided for disinfection, such as disinfection of the transmission component and/or parts thereof, such as the device cable or part thereof. The UV light emitter and/or the plurality of UV light emitters may be accommodated in the cable reel housing. In some examples, the UV light emitter may be arranged inside the spool. The UV light emitter and/or the plurality of UV light emitters may be activated based on whether the portion of the device cable is rolled onto the spool. For example, the UV light emitter and/or the plurality of UV light emitters may be activated in response to and/or after the portion of the device cable has been reeled in by the cable reel, e.g. in response to and/or after the portion of the device cable has been reeled onto the spool.

The transmission component may comprise a counter. The counter may be indicative of the number of times the transmission component has been used and/or may be indicative of deterioration of the transmission component and/or parts thereof. For example, the counter may be indicative of deterioration of the device cable and/or the component coupling part. The counter may be an electronically readable counter, e.g. a flash memory or other suitable electronic memory known in the art. Alternatively or additionally, the counter may be a physical wear indicator, e.g. an indicator changing color over time and/or based on UV light exposure.

The transmission component, such as the device cable and/or the component coupling part, may be coated with silver. Thereby, an antimicrobial effect may be achieved. Using a silver coating may be advantageous both independently as well as in combination with UV disinfection as described above. Furthermore, silver coating may be beneficial for the transmission component both when utilizing wireless communication with the video processing apparatus as well as when utilizing wired communication using the device cable.

The component coupling part of the transmission component may have a primary coupling element. The primary coupling element may be elongated and/or may extend along a coupling part axis. The coupling part axis may be perpendicular to the cable direction, as mentioned above. The device coupling part of the endoscope may have a secondary coupling element. The secondary coupling element may form a cavity, e.g. along the handle axis. The secondary coupling element, such as the cavity of the secondary coupling element, may be adapted to receive the primary coupling element, e.g. by a linear movement along the coupling part axis and/or the handle axis.

The device coupling part, such as the secondary coupling element, may comprise a twist lock. The twist lock may be adapted to engage by rotation of the handle with a protruding element of the component coupling part, e.g. of the primary coupling element, when the primary coupling element is fully received by the secondary coupling element. The rotation of the handle may be around the coupling part axis and/or the handle axis. The protruding element of the component coupling part may be the stiffened portion of the device cable.

The device coupling part, such as the secondary coupling element, may comprise one or more device terminals. The one or more device terminals may be electronically connected to the camera module and/or the light emitter. The component coupling part, such as the primary coupling element may comprise one or more component terminals. The one or more component terminals may be adapted to connect to the one or more device terminals of the device coupling part when the component coupling part is coupled with the device coupling part. Thereby, an electrical connection may be obtained between the camera module and/or the light emitter and the transmission component, such as between the camera module and/or the light emitter and the component processor of the transmission component.

As mentioned above, the present disclosure also comprises a support bracket for a medical visualization system, such as the disclosed medical visualization system. Accordingly, the medical visualization system may comprise the support bracket. The support bracket may have a distal support bracket part and a proximal support bracket part. The support bracket may extend between the distal support bracket part and the proximal support bracket part. The distal support bracket part may include a coupling part retainer. The coupling part retainer may be adapted to retain the component coupling part of the transmission component. In some examples, the support bracket may comprise the cable reel.

The coupling part retainer may comprise a switch, e.g. a mechanical switch (e.g. a button) or a hall switch. The switch of the coupling part retainer may be activated/deactivated based on the receipt of the component coupling part. The switch of the coupling part retainer may cause activation of the cable reel, such as to reel in or unreal the portion of the device cable. For example, the switch of the coupling part retainer may generate the first control input to the actuator of the cable reel to reel in the portion of the device cable, e.g. when the component coupling part is received in the coupling part retainer and/or the switch of the coupling part retainer may generate the second control input to the actuator of the cable reel to unreel the portion of the device cable, e.g. when the component coupling part is not received in and/or is removed from the coupling part retainer. In other word, the first control input may be triggered by the component coupling part being received by the coupling part retainer, e.g. the switch of the coupling part retainer may be subjective to receipt of the component coupling part and the switch may trigger the first control input. Additionally or alternatively, the second control input may be triggered by the component coupling part being removed from the coupling part retainer, e.g. the switch of the coupling part retainer may be subjective to removal of the component coupling part and the switch may trigger the second control input.

The proximal support bracket part may be adapted to be coupled to the video processing apparatus, such as to the housing of the video processing apparatus. For example, the proximal support bracket part may be adapted to be coupled to the video processing apparatus, such as to the housing of the video processing apparatus, such that the support bracket follows rotation of the housing around a first axis and/or such that the support bracket does not follow rotation of the housing around a second axis. The second axis may be perpendicular to the first axis. The first axis may be vertical. The second axis may be horizontal.

The video processing apparatus may comprise a mounting assembly. The mounting assembly may be adapted to attach the video processing apparatus, such as the housing of the video processing apparatus, to a mount, e.g. a stand, such as an IV pole. The mounting assembly may have a fastener, such as a clamp or similar. The fastener may be adapted to attach the video processing apparatus, such as the housing of the video processing apparatus, to the mount. The mounting assembly may have first hinge and/or a second hinge. The first hinge may be rotatable around a first axis (e.g. the same first axis as mentioned in the previous paragraph). The second hinge may be rotatable around a second axis (e.g. the same second axis as mentioned in the previous paragraph). The second axis may be perpendicular to the first axis. The first axis may be vertical. The second axis may be horizontal. The first hinge may be arranged between the fastener and the second hinge. The second hinge may be arranged between the first hinge and the housing of the video processing apparatus. The proximal support bracket part may be coupled to the mounting assembly between the first hinge and the second hinge.

The coupling part retainer may be adapted to receive and/or supply the component coupling part by linear movement of the component coupling part along a third axis. The component coupling part may be adapted to engage with and/or be removed from the coupling part retainer by linear movement of the component coupling part along the third axis. The third axis may be perpendicular to the first and/or second axis of any of the preceding paragraphs.

The coupling part axis may be non-parallel with the third axis, when the component coupling part is received by the coupling part retainer. For example, the third axis and the coupling part axis may form an angle, such as an angle between 45-90 degrees. For example, the third axis and the coupling part axis may be perpendicular.

The coupling part retainer and/or the component coupling part may be adapted to prevent rotation, e.g. at least around the first axis, the second axis and/or the third axis, of the component coupling part, e.g. relative to the coupling part retainer, when the component coupling part is received by the coupling part retainer.

The coupling part retainer and/or the component coupling part may be adapted to prevent movement, such as linear movement of the component coupling part, along the first axis and/or the second axis, when the component coupling part is received by the coupling part retainer.

The support bracket may comprise a support cable. The support cable may be extending between the distal support bracket part and the proximal support bracket part. The support cable may have a first end. The first end of the support cable may be arranged at the proximal support bracket part. The first end of the support cable may be adapted to connect to the video processing apparatus. For example, the first end of the support cable may comprise a connector. The support cable may have a second end. The support cable may extend from the first end to the second end. The second end of the support cable may be arranged at the distal support bracket part. The second end of the support cable may be adapted to connect to the transmission component. The support cable may be an integrated cable. For example, the support cable may be extending inside the support bracket.

The endoscope may be a laryngoscope, a broncoscope, a duodenoscope, a urology endoscope, or other type of endoscope known in the art.

The endoscope may comprise an insertion cord extending from the handle to a distal cord portion. The view from the endoscope may be a view from the distal cord portion of the insertion cord. The handle may comprise a control mechanism adapted to receive an input in a first input direction. The input in the first input direction may cause a bendable section of the insertion cord to bend in a first bending direction. The control mechanism may be adapted to receive an input in a second input direction, e.g. opposite the first input direction. The input in the second input direction may cause the bendable section of the insertion cord to bend in a second bending direction, e.g. opposite the first bending direction. In some examples, the control mechanism may be adapted to receive an input in a third input direction and/or to receive an input in fourth input direction, which respectively may cause the bendable section of the insertion cord to bend in a third and fourth bending direction, e.g. perpendicular to the first and/or second bending direction. Alternatively, the endoscope may comprise a second control mechanism adapted to receive the input in the third and/or fourth input directions.

The camera module of the endoscope may comprise an image sensor, such as. a CCD or a CMOS, to generate the image data indicative of the view from the endoscope. The endoscope may further comprise a light emitter, e.g. adapted to provide illumination of the view. The light emitter may be an LED, an optical fiber connectable to a light source, or similar element known to provide illumination. The camera module may include the light emitter. The camera module and/or the light emitter may be provided at a distal end of an insertion cord of the endoscope.

The endoscope may be disposable. The transmission component may be reusable. The transmission component may be adapted to be coupled to a plurality of endoscopes, such as a plurality of different types of endoscopes.

The video processing apparatus may comprise the display. The display may be accommodated in the housing of the video processing apparatus. Alternatively or additionally, the display may be an external display coupled to the video processing apparatus, such as to allow the video processing apparatus to cause it to display the live representation of the image data.

### BRIEF DESCRIPTION OF THE FIGURES

Embodiments of the disclosure will be described in more detail in the following with regard to the accompanying figures. The figures show one way of implementing the present disclosure and are not to be construed as being limiting to other possible embodiments falling within the scope of the attached claim set.
Fig. 1a schematically illustrates an exemplary medical visualization system,
Fig. 1b is a block diagram schematically illustrating an exemplary video processing apparatus,
Fig. 2a is a block diagram schematically illustrating an exemplary transmission component,
Fig. 2b is a block diagram schematically illustrating an exemplary endoscope,
Fig. 3a is a block diagram schematically illustrating an exemplary transmission component,
Fig. 3b is a block diagram schematically illustrating an exemplary endoscope,
Fig. 4 schematically illustrates an exemplary component coupling part,
Fig 5 schematically illustrates an exemplary transmission component and an exemplary endoscope,
Fig. 6 schematically illustrates a top view of an exemplary transmission component and an exemplary endoscope,
Fig. 7 schematically illustrates an exemplary mounting assembly,
Fig. 8 schematically illustrates an exemplary support bracket and a component coupling part,
Fig. 9 schematically illustrates an exemplary medical visualization system,
Fig. 10a schematically illustrates an exemplary medical visualization system, and
Fig. 10b schematically illustrates internal parts of an exemplary cable reel.

### DETAILED DESCRIPTION

Various exemplary embodiments and details are described hereinafter with reference to the figures when relevant. It should be noted that the figures may or may not be drawn to scale and that elements of similar structures or functions are represented by like reference numerals throughout the figures. It should also be noted that the figures are only intended to facilitate the description of the embodiments. They are not intended as an exhaustive description of the invention or as a limitation on the scope of the invention. In addition, an illustrated embodiment need not have all the aspects or advantages shown. An aspect or an advantage described in conjunction with a particular embodiment is not necessarily limited to that embodiment and can be practiced in any other embodiment even if not so illustrated, or if not so explicitly described.

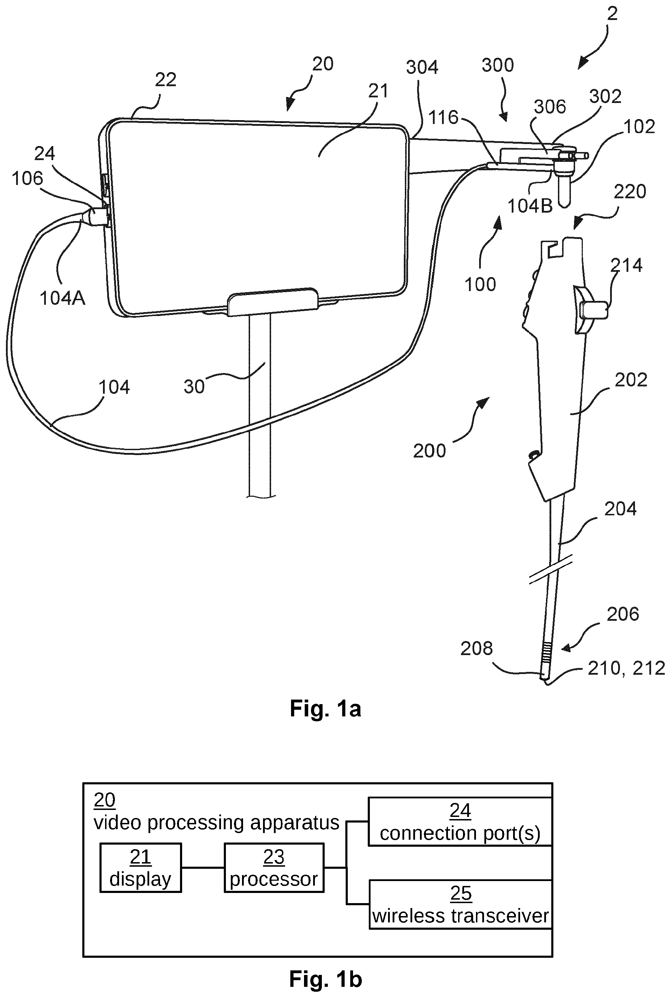

Fig. 1a schematically illustrates an exemplary medical visualization system 2 comprising an endoscope 200 and a video processing apparatus 20. The endoscope 200 has a camera module 210, e.g. including an image sensor, such as. a CCD or a CMOS. The camera module 210 is configured to generate image data indicative of a view from the endoscope 200. In the illustrated example, the endoscope 200 comprises a handle 202 and an insertion cord 8 extending from the handle 202 to a distal cord portion 208. The camera module 210 may be adapted to generate image data indicative of a view from the distal cord portion 208 of the insertion cord 8. The endoscope 200 may further comprise a light emitter 212 adapted to provide illumination of the view. The light emitter 212 may be an LED, an optical fiber connectable to a light source, or similar element known to provide illumination. In some examples, the light emitter 212 may form part of the camera module 210.

The visualization system 2 comprises a transmission component 100 adapted to transmit the image data and/or data representative of the image data to the video processing apparatus 20. Thereby, the endoscope 200 may be connected to the video processing apparatus 20. In the illustrated example, the transmission component 100 comprises a device cable 104. The device cable 104 extends from a first cable end 104A to a second cable end 104B. The device cable 104, in the illustrated example, terminates in a device connector 106 connected to a connection port 24 of the video processing apparatus 20. The device connector 106 is provided at the first cable end 104A.

The transmission component 100 comprises a component coupling part 102, which is adapted to couple with the endoscope 200, such as with a device coupling part 220 of the endoscope. The component coupling part 102 is provided at the second cable end 104B. Thus, in the present example, the device cable 104 is extending from the device connector 106 to the component coupling part 102.

Thus, in the present example, when the component coupling part 102 is coupled with the device coupling part, the transmission component 100 extends from the handle 202 of the endoscope 200 to the connection port 24 of the video processing apparatus.

The transmission component 100, such as the device cable 104 and/or the component coupling part 102, may be coated with silver to obtain an antimicrobial surface.

The device cable 104 comprises a stiffened portion 116 extending from the second cable end 104B and/or from the component coupling part 102 towards the first cable end 104A. The stiffened portion may have a length of between 20-150 mm from the second cable end 104B.

The video processing apparatus 20 is operable to receive the image data generated by the camera module 210 of the endoscope 200. For example, the video processing apparatus 20 may receive image data generated by the camera module 210 via the device coupling part 220, the device coupling part 102, the device cable 104, the connector 106 and the connection port 24.

The video processing apparatus 20 is configured to display images received from the endoscope 200. The video processing apparatus 20 comprises a housing 22. The video processing apparatus 20 may comprise a display 21, such as a touch sensitive display, as illustrated. Preferably, the display 21 is accommodated in the housing 22 of the video processing apparatus 20, as illustrated. Alternatively, or additionally, the video processing apparatus 20 may be connected to an external display where the images received from the endoscope 200 are displayed.

The handle 202 comprises a control mechanism 214 adapted to receive an input (e.g. a touch input) in a first input direction and/or in a second input direction. The input in the first input direction on the control mechanism 214 causes a bending section 206 of the insertion cord 204 to bend in a first bending direction, e.g. via wires extending from the handle 202, through the insertion cord 204 to the bending section 206. The input in the second input direction on the control mechanism 214 causes the bending section 206 of the insertion cord 204 to bend in a second bending direction. The first input direction and the second input direction may be opposite. The first bending direction and the second bending direction may be opposite. Bending the bending section 206 of the insertion cord 204 may cause a movement of the distal cord portion 208 and the camera module 210 in a direction relative to the camera module 210. Thereby, seeing an image generated by the camera module 210, a direction, e.g. up or down, in the image may correspond to a respective input on the control mechanism 214. In some examples, the endoscope 200 may additionally provide the possibility of also bending the bending section 206 in non-parallel direction, e.g. in a perpendicular direction. This additional non-parallel or perpendicular bending may be provided for by the control mechanism 214 or by an additional control mechanism. Hence, the bending section may in such case cause movement of the distal cord portion 208 in four directions, e.g. left/right and up/down.

The endoscope 200 may be disposable, e.g. single use. Conversely, the transmission component 100 may be reusable. Thus, the transmission component 100 may be adapted to be coupled to a plurality of endoscopes, such as a plurality of different types of endoscopes, e.g. including bronchoscopes, laryngoscopes, duodenoscopes, urology endoscopes etc.

The medical visualization system 2 further comprises an optional support bracket 300. The support bracket 300 has a distal support bracket part 302 and a proximal support bracket part 304. The support bracket 300 extends between the distal support bracket part 302 and the proximal support bracket part 304. The support bracket 300 comprises a coupling part retainer 306. The coupling part retainer 306 is adapted to retain the component coupling part 102, as illustrated. In the illustrated example, the distal support bracket part 302 includes the coupling part retainer 306. The proximal support bracket part 304 is coupled to video processing apparatus 20, such as the housing 22 of the video processing apparatus 20. In some examples, the support bracket 300 may follow rotation of the housing 22 around a first axis, e.g. a vertical axis, e.g. while not following rotation of the housing 22 around a second axis perpendicular to the first axis, e.g. a horizontal axis.

Fig. 1b is a block diagram schematically illustrating an exemplary video processing apparatus 20, such as the video processing apparatus 20 of Fig. 1a. The video processing apparatus 20 comprises a processor 23 adapted to receive the image data and/or data representative of the image data, e.g. via the connection port 24, as explained above. The processor 23 is further adapted to cause a display, e.g. the optional display 21 of the video processing apparatus 20, to display a live representation of the image data. The video processing apparatus 20 may also or alternatively comprise an apparatus wireless transceiver 25. Accordingly, the processor 23 may be adapted to receive the image data and/or data representative of the image data via the apparatus wireless transceiver 25, which will be explained in further detail below.

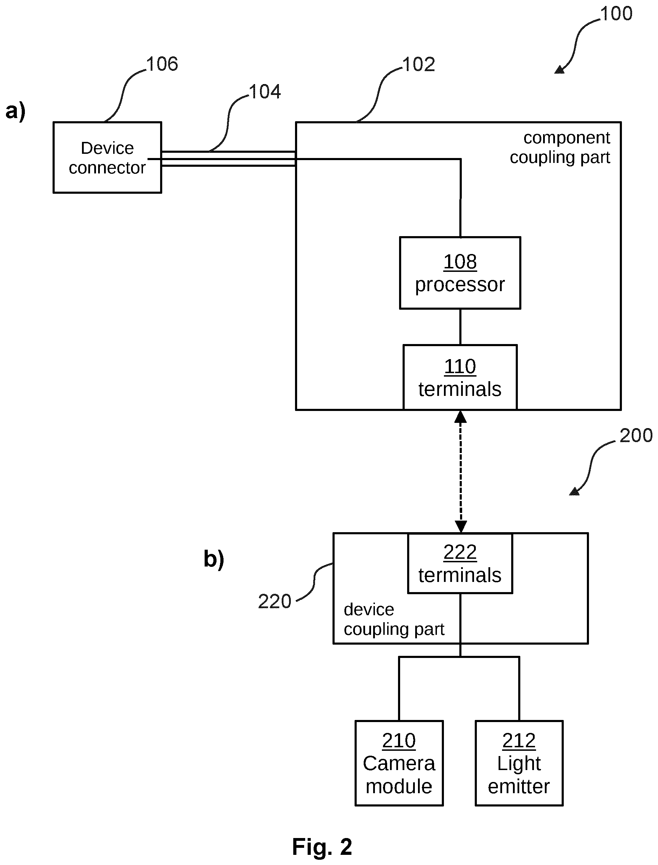

Fig. 2a is a block diagram schematically illustrating an exemplary transmission component 100, with a component coupling part 102, a device cable 104, and a device connector 106. Fig. 2b is a block diagram schematically illustrating an exemplary endoscope 200 with a camera module 210 and an optional light emitter 212.

The transmission component 100 comprises a component processor 108, which is adapted to receive the image data from the camera module 210 and preprocess the image data to provide data representative of the image data, which may then be transmitted to the video processing apparatus, e.g. via the device cable 104 and the device connector 106. The component coupling part 102 may comprise the component processor 108, as illustrated.

The transmission component 100 comprises one or more component terminals 110 adapted to connect to one or more device terminals 222 of the device coupling part 220 when the component coupling part 102 is coupled with the device coupling part 220. The component coupling part 102 may comprise the one or more component terminals 110. The component terminals 110 may form an electrical connection between the transmission component 100 and the endoscope 200, such as between the component coupling part 102 and the device coupling part 220. The component terminals 110 may be connected with the component processor 108 and may facilitate an electrical connection between the component processor 108 and the camera module 210 of the endoscope, i.e. facilitating the receipt of the image data generated by the camera module 210.

The endoscope 200 may be the endoscope 200 as illustrated in Fig. 1a. The endoscope 200 comprises a device coupling part 220. The device coupling part 220 is adapted to be coupled with the component coupling part 102. In the illustrated example, the device coupling part 220 comprises one or more device terminals 222 electrically connected to the camera module 210 and optionally to the light emitter 212. The device terminals 222 may facilitate an electrical connection between the component processor 108 of the transmission component 100 and the camera module 210, i.e. facilitating the transmission of the image data generated by the camera module 210.

In some examples, the transmission component 100 may facilitate transmission of data and/or signals from the video processing apparatus to the endoscope 200, such as to the camera module 210 and/or the light emitter 212. For example, camera configuration data may be transmitted from the video processing apparatus to the endoscope 200 / camera module 210, e.g. via the transmission component 100, such as via the device connector 106, the device cable 104 and the component coupling part 102.

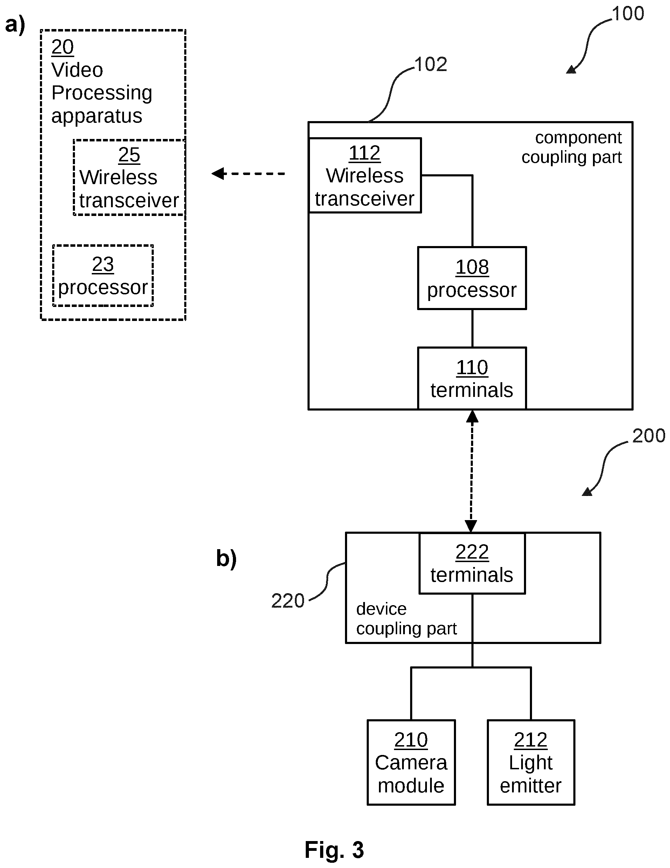

Fig. 3a is a block diagram schematically illustrating an exemplary transmission component 100 with a component coupling part 102. However, instead of the device cable 104 and the device connector 106, as illustrated in Fig. 2a, the transmission component 100 of Fig. 3a comprises a device wireless transceiver 112. Fig. 3b corresponds to Fig. 2b. The device wireless transceiver 112 is adapted to wirelessly communicate with an apparatus wireless transceiver 25 of the video processing apparatus 20, such as the video processing apparatus 20 as illustrated in Fig. 1a. The apparatus wireless transceiver 25 may be an integrated element of the video processing apparatus 20. However, in other examples, the apparatus wireless transceiver 25 may be connected to the video processing apparatus, in a similar way as the device connector 106, as illustrated in Fig. 1a. The device wireless transceiver 112 is adapted to wirelessly transmit the image data and/or the data representative of the image data to the video processing apparatus 20, e.g. to the apparatus wireless transceiver 25 of the video processing apparatus 20.

In the present disclosure a "transceiver" is meant as being either or both of a transmitter and a receiver. Thus, in one example, the device wireless transceiver 112 may be a transmitter and the apparatus wireless transceiver 25 may be a receiver. In another example, the device wireless transceiver 112 comprises both a transmitter and a receiver and/or the apparatus wireless transceiver 25 comprises both a transmitter and a receiver.

Like above, the transmission component 100 may, also in the present example, comprise a wireless transceiver 112 and facilitate transmission of data and/or signals from the video processing apparatus to the endoscope 200, such as to the camera module 210 and/or the light emitter 212. For example, camera configuration data may be transmitted from the video processing apparatus to the endoscope 200 / camera module 210, e.g. via the transmission component 100, such as via the device wireless transceiver 112.

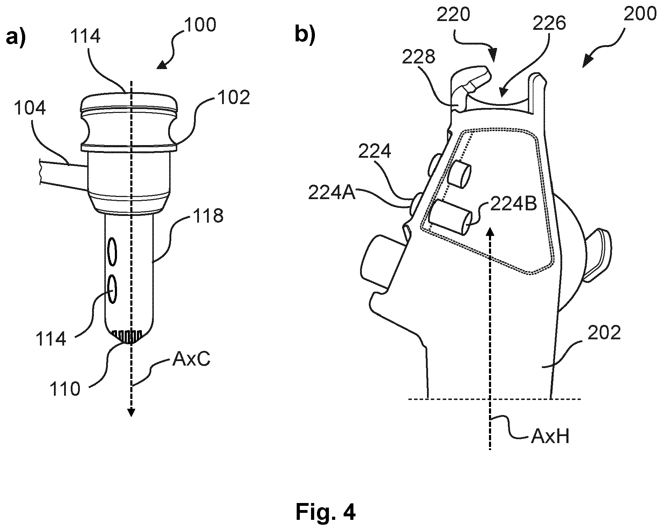

Fig. 4 schematically illustrates an exemplary component coupling part 102 of an exemplary transmission component 100 (Fig. 4a) and an exemplary device coupling part 220 of an exemplary endoscope 200 (Fig. 4b). In the illustrated example, an outer shell part of the endoscope 200 is removed to show some internal parts of the device coupling part 220.

The component coupling part 102 has a primary coupling element 118. The primary coupling element is elongated and extends along a coupling part axis AxC. The device coupling part 220 has a secondary coupling element 226. The secondary coupling element 226 forms a cavity adapted to receive the primary coupling element 118. The handle 202 is an elongate element extending along the handle axis AxH. The cavity of the secondary coupling element 226 may extend along a handle axis AxH of the handle 202.

The device coupling part 220 comprises a twist lock 228. The twist lock is adapted to engage by rotation of the handle 202, e.g. around the coupling part axis AxC and/or the handle axis AxH, with a protruding element of the component coupling part 102, e.g. of the primary coupling element 118, when the primary coupling element 118 is fully received by the secondary coupling element 226. In the illustrated exemplary embodiments, the protruding element engaging with the twist lock may be a part of the device cable 104

As illustrated in Fig. 4, the component coupling part 102 comprises one or more physical activatable input mechanisms 114. Activation of the one or more physical activatable input mechanisms 114 causes the transmission component 100 to transmit one or more input mechanism signals to the video processing apparatus indicative of activation of the one or more physical activatable input mechanisms 114. Accordingly, a user may activate a function of the video processing apparatus 20 (see Fig. 1a) by activation of one of the physical activatable input mechanisms 114. In some examples, a physical activatable input mechanism 114 may be a button, e.g. a button directly depressible by the user, such as the top part of the component coupling part 102. In other examples, a physical activatable input mechanisms 114 may be a lever, a switch or similar.

According to the invention, the one or more physical activatable input mechanisms 114 are provided on a part received by the device coupling part 220, when the component coupling part 102 is connected with the device coupling part 220. The handle 202 of the endoscope 4, such as the device coupling part 220, comprises one or more communicator elements 224. The one or more communicator elements 224 may be plungers, levers etc. The one or more communicator elements 224 is adapted to activate the one or more physical activatable input mechanisms 114, when being manipulated by a user. For example, as illustrated, the communicator elements 224 may extend from a first side 224A exteriorly of the handle 202 to a second side 224B interiorly of the handle 202. The second side 224B is adapted to contact a respective physical activatable input mechanism of the one or more physical activatable input mechanisms 114.

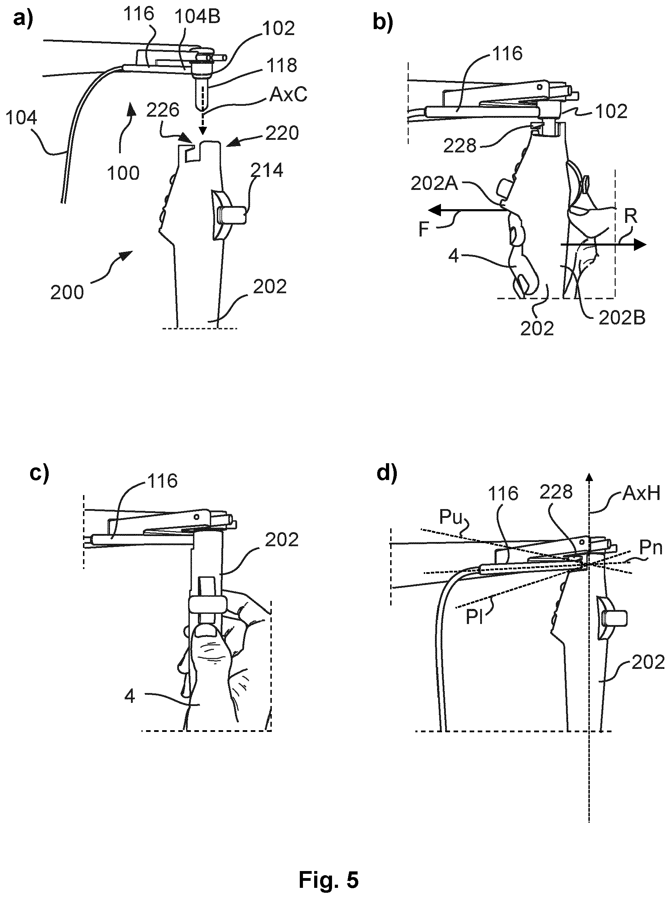

Figs 5a-d schematically illustrate an exemplary transmission component 100 with a component coupling part 102 and an exemplary endoscope 200 with a device coupling part 220. Figs. 5a-d further illustrate an exemplary connection between the component coupling part 102 and the device coupling part 220.

The device cable 104 comprises a stiffened portion 116 extending from the second cable end 104B. The stiffened portion may be substantially straight. Alternatively, the stiffened portion may be curved either upwards and/or away from the operator. As best seen in Fig. 5d, when the component coupling part 102 is connected with the device coupling part 220, the stiffened portion 116 of the device cable 104 may extend in a certain direction relative to the handle 202, such as relative to the handle axis AxH. For example, the stiffened portion 116 of the device cable 104 may extend in a normal plane Pn normal to the handle axis AxH, as illustrated. In some examples, the stiffened portion 116 may extend at an angle relative to the normal plane Pn normal to the handle axis AxH, and/or the stiffened portion 116 may be curved upwards or downwards. In such examples, the stiffened portion 116 may extend between an upper plane Pu and a lower plane PI. The upper plane Pu may form an angle of less than 15 degrees, e.g. +15 degrees, e.g. between 0 and 15 degrees, relative to the normal plane Pn. The lower plane PI may form an angle of less than 15 degrees, e.g. -15 degrees, e.g. between -15 and 0 degrees, relative to the normal plane Pn. The lower plane PI may form an angle of 30 degrees relative to the upper plane Pu. The lower plane PI, the upper plane Pu and the normal plane Pn may intersect the handle axis AxH at the same point.

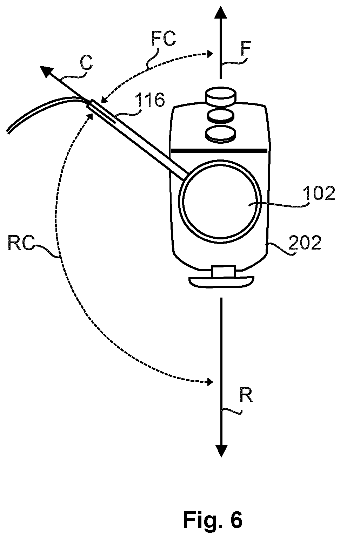

As best seen in Figs. 5b and 5c, the handle 202 is adapted to be gripped by a first hand 4 of an operator such that a back side 202B of the handle 202 is facing in a backwards direction R towards the wrist of the first hand 4 and a front side 202A is facing in a forward direction F opposite the backward direction R. The first hand may be a left hand or a right hand. For illustrative purposes, the figures show a right hand gripping the handle 202, although it may be more customary to grip an endoscope with the left hand. When the component coupling part 102 is connected with the device coupling part 220, as illustrated in Fig. 5d, the stiffened portion 116 may extend in a cable direction between the forward direction F and the backwards direction R. Fig. 6 schematically illustrates a top view of the arrangement illustrating that the stiffened portion 116 extends in a cable direction C, and that the cable direction C is between the forward direction F and the backwards direction R. The cable direction C may be perpendicular to the coupling part axis AxH (see Fig. 4). An angle FC between the cable direction C and the forward direction F may be less than 90 degrees, such as between 5 and 85 degrees, e.g. 60 degrees or 45 degrees. An angle RC between the cable direction C and the backward direction R may be more than 90 degrees, such as between 95 degrees and 175 degrees, e.g. 120 degrees or 135 degrees.

Turning again to Figs. 5a-5d, to attach the endoscope 200 to the transmission component 100, the primary coupling element 118 is inserted into the secondary coupling element 226, along the coupling part axis AxC and the handle axis AxH. In other words, the elongate element of the primary coupling element 118 is inserted into the cavity of the secondary coupling element 226. This is seen in Figs. 5a-5b.

The device coupling part 220, e.g. the secondary coupling element 226, comprises a twist lock 228 adapted to engage by rotation of the handle 202, e.g. around the coupling part axis AxC and/or the handle axis AxH, with a protruding element of the component coupling part 102, e.g. of the primary coupling element 118. In the exemplary embodiments, the protruding element is the stiffened portion 116 of the device cable 104. As seen in Figs. 5c-5d, the handle 202 is twisted to align the stiffened portion 116 with opening of the twist lock (Fig. 5c), and after the primary coupling element 118 is fully received (along the coupling part axis AxC and/or along the handle axis AxH) by the secondary coupling element 226, the handle is twisted (e.g. clockwise or counterclockwise) to lock the device coupling part 220 and the component coupling part 102.

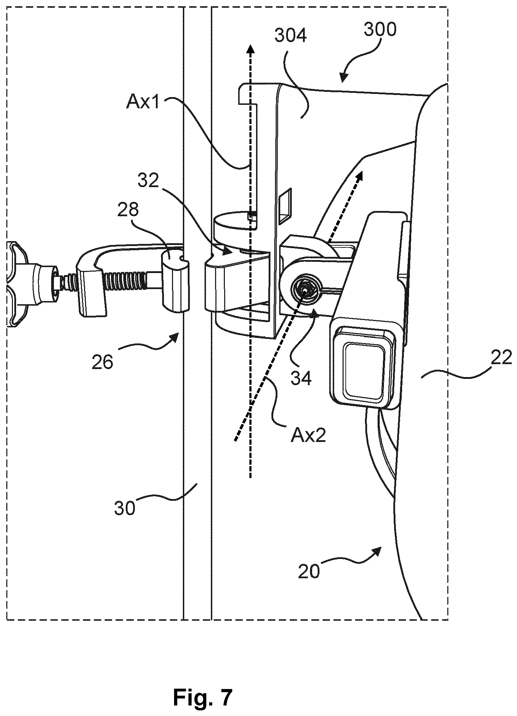

Fig. 7 schematically illustrates an exemplary mounting assembly 26 adapted to attach the housing 22 of the video processing apparatus 20 and the support bracket 300 to a mount 30, e.g. a stand, such as an IV-pole. The video processing apparatus 22 may comprise the mounting assembly 26.

The mounting assembly 26 has a fastener 28, e.g. a clamp, adapted to attach the video processing apparatus 20 to a mount 30. The mounting assembly 26 comprises a first hinge 32 rotatable around a first axis Ax1, e.g. vertically, and a second hinge 34 rotatable around a second axis Ax2, e.g. horizontally. The first axis Ax1 and the second axis Ax2 may be perpendicular. The first hinge 32 is arranged between the fastener 28 and the second hinge 34. The second hinge 34 is arranged between the first hinge 32 and the housing 22 of the video processing apparatus 20. The proximal support bracket part 304 is coupled to the mounting assembly 26 between the first hinge 32 and the second hinge 34. Thereby, the support bracket 300 follows the rotation of the first hinge 32, i.e. around the first axis Ax1, but do not follow the rotation of the second hinge 34, i.e. around the second axis Ax1. Thus, tilting the housing 22 of the video processing apparatus 20 will not affect the orientation of the support bracket 300. On the other hand, turning the housing 22 affects the orientation of the support bracket 300 correspondingly.

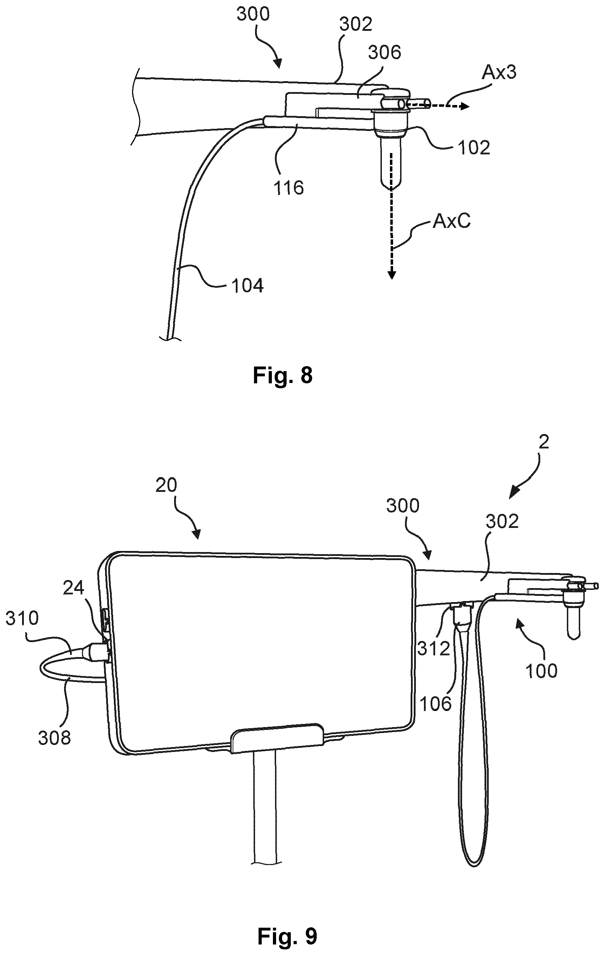

Fig. 8 schematically illustrates an exemplary support bracket 300 and the component coupling part 102 retained by the support bracket 300. As seen the distal support bracket part 302 includes the coupling part retainer 306, which is adapted to retain the component coupling part 102.

The coupling part retainer 306 is adapted to receive and/or supply the component coupling part 102 by linear movement of the component coupling part 102 along a third axis Ax3. Similarly, the component coupling part 102 is adapted to engage with the coupling part retainer 306 by the linear movement along the third axis Ax3. The coupling part axis AxC may be non-parallel with the third axis Ax3, when the component coupling part 102 is received by the coupling part retainer 306. For example, the coupling part axis AxC and the third axis Ax3 may form an angle between 45-90 degrees. For example, the coupling part axis AxC and the third axis Ax3 may be perpendicular.

The coupling part retainer 306 and/or the component coupling part 102 may be adapted to prevent rotation of the component coupling part 102 relative to the coupling part retainer 306, when the component coupling part 102 is received by the coupling part retainer 306. To prevent rotation, the part of the component coupling part 102 engaging with the coupling part retainer 306 may have a non-circular shape, e.g. an oval shape or an elliptical shape.

The coupling part retainer 306 and/or the component coupling part 102 may be adapted to prevent movement, such as linear movement, of the component coupling part 102 along the coupling part axis AxC and/or along the first axis Ax1 and/or the second axis Ax2 (see Fig. 7), when the component coupling part 102 is received by the coupling part retainer 306. To prevent movement, such as linear movement, of the component coupling part 102 along the coupling part axis AxC (and/or along the first axis Ax1 and/or the second axis Ax2), the part of the component coupling part 102 engaging with the coupling part retainer 306 may have a concave shape.

In the present example, the user may couple the handle 202 of the endoscope 200 to the component coupling part 102, as explained in relation to Figs. 5a-5d, and subsequently remove the component coupling part 102 (while being attached to the handle 202) from the coupling part retainer 306 by moving the handle linearly away from the coupling part retainer 306 along the third axis Ax3 (Fig. 8). Similarly, e.g. after use, the user may return the component coupling part 102 (while being attached to the handle 202) to the coupling part retainer 306 by aligning the component coupling part 102 and the coupling part retainer 306, and position the component coupling part 102 in the coupling part retainer 306 by movement along the third axis Ax3. The user may let both the component coupling part 102 and the attached endoscope 200 be held by the coupling part retainer 306, as seen in Fig. 5d. To decouple the endoscope 200 from the component coupling part 102, the user may turn the handle 202, as illustrated in Fig. 5c, and remove the handle 202 and the endoscope 200 from the component coupling part 102 along the coupling part axis AxC. Thus, by the present disclosure, both coupling and decoupling of the endoscope 200 and component coupling part 102 may be performed without a need to touch the component coupling part 102 or the transmission component 100, which may be reusable. Thereby the risk of cross contamination may be reduced.

Fig. 9 schematically illustrates an exemplary medical visualization system 2 like the medical visualization system 2 of Fig. 1a. However, in Fig. 9, the support bracket 300 comprises a support cable 308. The support cable 308 extends (e.g. inside the support bracket) between the distal support bracket part 302 and the proximal support bracket part 304. A first end 310 of the support cable 308, e.g. being arranged at and/or extending from the proximal support bracket part 304, is adapted to connect to the video processing apparatus 20, as illustrated. A second end 312 of the support cable 308, e.g. being arranged at the distal support bracket part 302, is adapted to connect to the transmission component 100. For example, as illustrated, the second end 312 of the support cable 308 may terminate in a connection port similar to the connection port 24 of the video processing apparatus 20, and the device connector 106 of the transmission component 100 may be connected with the connection port formed by the second end 312 of the support cable 308.

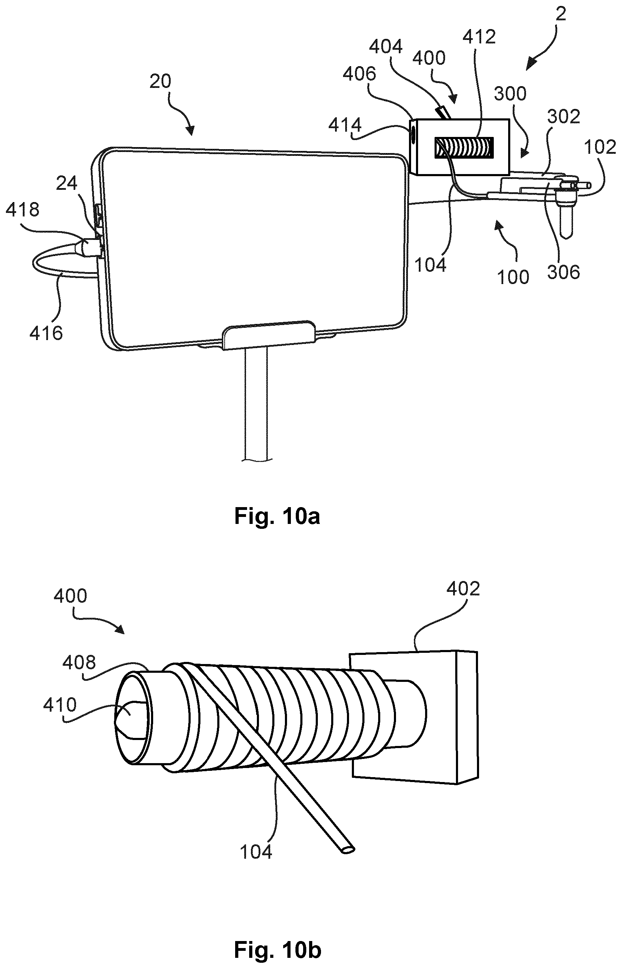

Fig. 10a schematically illustrates an exemplary medical visualization system 2 like the medical visualization system 2 of Fig. 1a or 9. However, in Fig. 10a, the medical visualization system 2 comprises a cable reel 400. Internal parts of the cable reel 400 is schematically illustrated in Fig. 10b. The cable reel 400 may comprise a cable reel housing 406, which is not shown in Fig. 10b, to allow visibility of the internal parts of the cable reel 400. Figs. 10a and 10b are described together in the following. The cable reel 400 is adapted to reel in at least a portion of the device cable 104. The cable reel housing 406 may enclose the portion of the device cable 104 when reeled in. In some examples, the cable reel 400 forms part of the support bracket 300. In some other examples, the cable reel 400 forms part of the transmission component 100. A connector cable 416 may extend from the cable reel 400 and may be adapted to connect to the video processing apparatus 20 at its first end 418. The connector cable 416 may be adapted to connect the video processing apparatus 20 and the device cable 104. In some examples, the connector cable 416 is the support cable 308 as previously described. In other examples, the connector cable 416 may form part of the cable reel 400. In further examples, the connector cable 416 is a separate cable adapted to connect the cable reel 400 to the video processing apparatus 20.

The cable reel 400 may be driven by an actuator 402. The actuator 402 may be a rotary actuator, e.g. an electrical motor, such as a DC motor. For example, the actuator 402 may drive the cable reel 400 such that the device cable 104 is reeled in and out as appropriate. For example, this may be advantageous so as to reduce any influence of the cable reel on the operator's ability to control the endoscope being attached to the device cable 104, e.g. by the component coupling part 102. The actuator may be controlled based on user inputs, e.g. via the video processing apparatus 20 (e.g. via touch screen input on the video processing apparatus), or via a dedicated input device 404 (e.g. a switch, button, lever or similar) of the cable reel 400. For example, a first position of the input device 404 may cause the cable reel 400 to reel in the device cable 104, and a second position of the input device 404 may cause the cable reel 400 to unreel the device cable 104. The actuator 402 may be activated to reel in the device cable 104 based on a first control input and/or may be activated to unreel the device cable 104 based on a second control input. The first and/or second control input may be an input from the input device 404 and/or from the video processing apparatus 20. In some examples, the input device 404 may be employed in the coupling part retainer 306. For example, the input device 404 may be implemented as a switch, e.g. a physical switch or a hall switch, detecting the presence of the component coupling part 102 being received in the coupling part retainer 306. Thereby, removal of the component coupling part 102 from the coupling part retainer 306 may cause the cable reel 400, e.g. the actuator 402 of the cable reel 400, to unreel the device cable 104. Alternatively or additionally receipt of the component coupling part 102 in the coupling part retainer 306 may cause the cable reel 400, e.g. the actuator 402 of the cable reel 400, to reel in the device cable 104.

The cable reel comprises a spool 408. The spool 408 is accommodated in the cable reel housing 406. The portion of the device cable 104 is rolled onto the spool 408 when reeled in by the cable reel 400. The spool 408 may be transparent to UV light. For example, the spool 408 may be made from Polymethylmethacrylate (PMMA).

The cable reel 400 may comprise a UV light emitter 410. The UV light emitter 410 is accommodated in the cable reel housing 406. In the illustrated example, the UV light emitter 410 is arranged inside the spool 408. Thereby, in combination with the spool 408 being transparent, the UV light emitter 410 is able to subject the part of the device cable 104 rolled onto the spool 408 to UV light. Alternatively or additionally, the device cable 104 may be transparent and/or partly transparent to UV light. For example, the device cable 104 may comprise a transparent material, e.g. polymer, enclosing the electrical conducing wires. Making the device cable 104 transparent to UV light reduces the risk of parts of the device cable 104 blocking other parts from exposure of the UV light, e.g. if the device cable 104 is coiled up over itself. Thereby, more efficient disinfection of more of the device cable 104 may be achieved.

To contain the UV light within the cable reel housing 406, the opening 412 through which the device cable 104 protrude may be made small or may be covered with bristles or similar to avoid or at least reduce UV light from escaping the cable reel housing 406. The cable reel housing 406 may be opaque to UV light. The UV light emitter 410 may be activated automatically, e.g. after the device cable 104 is rolled onto the spool 408. Alternatively or additionally, the UV light emitter 410 may be activated by a user input, e.g. via the video processing apparatus 20 (e.g. via touch screen input on the video processing apparatus), or via a dedicated second input device 414 (e.g. a switch, button, lever or similar) of the cable reel 400. It may also be envisioned that the opening 412 may be closable, e.g. the opening 412 may comprise a shutter for closing the opening 412. In such an example, activation of the UV light emitter 410 may be activated based on whether the opening 412 is closed. For example, the UV light emitter 410 may be only activated if the opening 412 is closed and/or the UV light emitter 410 may be activated automatically when the opening 412 is closed.

In some examples, the transmission component 100 comprises a counter. The counter may be indicative of the number of times the transmission component 100 has been used and/or may be indicative of the number of times the transmission component 100 (such as the device cable 104) has been subjected to UV light by the UV light emitter 410 and/or may be indicative of deterioration of the transmission component 100. The counter may be an electronically readable counter, e.g. a flash memory or other suitable electronic memory known in the art. The electronically readable counter may be readable by the video processing apparatus 20 which may then indicate to the user the value of the counter, and/or indicate to the user whether it is time to replace the transmission component 100. Alternatively or additionally, the counter may be employed by providing the transmission component with a physical wear indicator, e.g. a part changing color over time and/or based on UV light exposure. The counter may be indicative of deterioration of the transmission component 100, such as of the device cable 104 of the transmission component 100, which may be especially relevant when using UV light for disinfection, because UV light can be harsh on materials used for the transmission component 100 such as materials used for the device cable 104.

The disclosure has been described with reference to a preferred embodiment. However, the scope of the invention is defined in the appended claims.

Throughout the description, the use of the terms "first", "second", "third", "fourth", "primary", "secondary", "tertiary" etc. does not imply any particular order or importance but are included to identify individual elements. Furthermore, the labelling of a first element does not imply the presence of a second element and vice versa.

### LIST OF REFERENCES

- 2: medical visualization system
- 4: hand
- 20: video processing apparatus
- 21: display
- 22: housing
- 23: processor
- 24: connection port(s)
- 25: apparatus wireless transceiver
- 26: mounting assembly
- 28: fastener
- 30: mount
- 32: first hinge
- 34: second hinge
- 100: transmission component
- 102: component coupling part
- 104: device cable
- 104A, 104B: ends of device cable
- 106: device connector
- 108: component processor
- 110: component terminal(s)
- 112: device wireless transceiver
- 114: input mechanisms
- 116: stiffened portion
- 118: primary coupling element
- 200: endoscope
- 202: handle
- 202A, 202B: sides of handle
- 204: insertion cord
- 206: bending section
- 208: distal cord portion
- 210: camera module
- 212: light emitter
- 214: control mechanism
- 220: device coupling part
- 222: device terminal(s)
- 224: communicator elements
- 224B, 224B: ends of communicator element(s)
- 226: secondary coupling element
- 228: twist lock
- 300: support bracket
- 302: distal support bracket part
- 304: proximal support bracket part
- 306: coupling part retainer
- 308: support cable
- 310: first end of support cable
- 312: second end of support cable
- 400: cable reel
- 402: actuator
- 404: input device
- 406: cable reel housing
- 408: spool
- 410: UV light emitter
- 412: opening
- 414: second input device
- 416: connector cable
- 418: first end of connector cable
- Ax1: first axis
- Ax2: second axis
- Ax3: third axis
- AxH: handle axis
- AxC: coupling part axis
- R: backward direction
- F: forward direction
- C: cable direction
- RC: angle between cable and backward directions
- FC: angle between cable and forward directions
- Pn: normal plane
- Pu: upper plane
- PI: lower plane

## Claims

1. A medical visualization system (2) comprising a video processing apparatus (20), an endoscope (200) and a transmission component (100),
the endoscope comprising a camera module (210) adapted to generate image data indicative of a view from the endoscope, a handle (202), and a device coupling part (220),
the video processing apparatus comprising a housing (22) and a processor (23) adapted to receive the image data and/or data representative of the image data and cause a display to display a live representation of the image data,
the transmission component comprising a component processor (108) adapted to receive the image data from the camera module and preprocess the image data to provide the data representative of the image data, the transmission component being adapted to transmit the data representative of the image data to the video processing apparatus, the transmission component comprising a component coupling part (102) adapted to couple with the device coupling part, wherein the component coupling part comprises the component processor,
wherein the component coupling part comprises one or more physical activatable input mechanisms (114),
and wherein activation of the one or more physical activatable input mechanisms causes the transmission component to transmit one or more input mechanism signals to the video processing apparatus indicative of activation of the one or more physical activatable input mechanisms, and
wherein the handle of the endoscope comprises one or more communicator elements (224)
**characterized in that** the communicator elements are
adapted to activate the one or more physical activatable input mechanisms upon being manipulated by a user.

2. Medical visualization system according to claim 1, wherein a first communicator element of the one or more communicator elements extends from a first side (224A) to a second side (224B), wherein the first side is arranged exteriorly of the handle and the second side is arranged interiorly of the handle, and wherein the second side is adapted to contact a first physical activatable input mechanism of the one or more physical activatable input mechanism when the component coupling part is coupled with the device coupling part.

3. Medical visualization system according to any one of the preceding claims, wherein the transmission component comprises a device cable (104) extending from a first cable end (104A) to a second cable end (104B), wherein the component coupling part is provided at the second cable end.

4. Medical visualization system according to claim 3, wherein the device cable comprises a stiffened portion (116) extending from the second cable end towards the first cable end, and optionally wherein the stiffened portion has a length of between 20-150 mm from the second cable end.

5. Medical visualization system according to any one of claims 3-4 comprising a cable reel (400) adapted to reel in at least a portion of the device cable.

6. Medical visualization system according to claim 5, wherein the cable reel comprises a cable reel housing (406) enclosing the portion of the device cable when reeled in, and optionally wherein the cable reel comprises a UV light emitter (410) accommodated in the cable reel housing.

7. Medical visualization system according to any one of the preceding claims, wherein the component coupling part has a primary coupling element (118) being elongated and extending along a coupling part axis, and wherein the device coupling part has a secondary coupling element (226) forming a cavity adapted to receive the primary coupling element.

8. Medical visualization system according to claim 7, wherein the device coupling part comprises a twist lock (228) adapted to engage by rotation of the handle with a protruding element of the component coupling part when the primary coupling element is fully received by the secondary coupling element.

9. Medical visualization system according to any one of the preceding claims further comprising a support bracket (300) having a distal support bracket part (302) and a proximal support bracket part (304), and wherein the distal support bracket part includes a coupling part retainer (306) adapted to retain the component coupling part of the transmission component.

10. Medical visualization system according to claim 9, wherein the proximal support bracket part is adapted to be coupled to the housing of the video processing apparatus, e.g. such that the support bracket follows rotation of the housing around a first axis and does not follow rotation of the housing around a second axis perpendicular to the first axis.

11. Medical visualization system according to any one of claims 9-10, wherein the coupling part retainer is adapted to receive the component coupling part by linear movement of the component coupling part along a third axis, and/or wherein the component coupling part is adapted to engage with the coupling part retainer by linear movement of the component coupling part along the third axis.

12. Medical visualization system according to claim 11 as dependent on at least claim 7, wherein the coupling part axis is non-parallel with the third axis, such as perpendicular to the third axis, when the component coupling part is received by the coupling part retainer.

13. Medical visualization system according to any one of claims 9-12 as dependent on at least claim 5, wherein the support bracket comprises the cable reel, the cable reel being driven by an actuator (402), and wherein the actuator is activated to reel in the portion of the device cable based on a first control input, wherein the first control input is triggered by the component coupling part being received by the coupling part retainer, e.g. the coupling part retainer comprises a switch subjective to receipt of the component coupling part and the switch triggers the first control input.

## Patentansprüche

1. Medizinisches Visualisierungssystem (2), das eine Videoverarbeitungseinrichtung (20), ein Endoskop (200) und eine Übertragungskomponente (100) umfasst,
wobei das Endoskop ein Kameramodul (210), das so angepasst ist, dass es Bilddaten erzeugt, die eine Sicht von dem Endoskop angeben, einen Griff (202) und ein Vorrichtungskopplungsteil (220) umfasst,
wobei die Videoverarbeitungseinrichtung ein Gehäuse (22) und einen Prozessor (23) umfasst, der so angepasst ist, dass er die Bilddaten und/oder Daten, die für die Bilddaten repräsentativ sind, empfängt und bewirkt, dass eine Anzeige eine Live-Darstellung der Bilddaten anzeigt,
wobei die Übertragungskomponente einen Komponentenprozessor (108) umfasst, der so angepasst ist, dass er die Bilddaten von dem Kameramodul empfängt und die Bilddaten verarbeitet, um die für die Bilddaten repräsentativen Daten bereitzustellen, wobei die Übertragungskomponente so angepasst ist, dass sie die für die Bilddaten repräsentativen Daten an die Videoverarbeitungseinrichtung überträgt, wobei die Übertragungskomponente ein Komponentenkopplungsteil (102) umfasst, das so angepasst ist, dass es mit dem Vorrichtungskopplungsteil koppelt,
wobei das Komponentenkopplungsteil den Komponentenprozessor umfasst, wobei das Komponentenkopplungsteil einen oder mehrere physisch aktivierbare Eingabemechanismen (114) umfasst, und
wobei die Aktivierung des einen oder der mehreren physisch aktivierbaren Eingabemechanismen bewirkt, dass die Übertragungskomponente ein oder mehrere Eingabemechanismussignale, die die Aktivierung des einen oder der mehreren physisch aktivierbaren Eingabemechanismen angeben, an die Videoverarbeitungseinrichtung überträgt, und
wobei der Griff des Endoskops ein oder mehrere Kommunikatorelemente (224) umfasst, **dadurch gekennzeichnet, dass** die Kommunikatorelemente so angepasst sind, dass sie, sobald sie von einem Benutzer manipuliert werden, den einen oder die mehreren physisch aktivierbaren Eingabemechanismen aktivieren.

2. Medizinisches Visualisierungssystem nach Anspruch 1, wobei sich ein erstes Kommunikatorelement des einen oder der mehreren Kommunikatorelemente von einer ersten Seite (224A) zu einer zweiten Seite (224B) erstreckt, wobei die erste Seite außen am Griff angeordnet ist und die zweite Seite innen im Griff angeordnet ist, und wobei die zweite Seite so angepasst ist, dass sie einen ersten physisch aktivierbaren Eingabemechanismus des einen oder der mehreren physisch aktivierbaren Eingabemechanismen berührt, wenn das Komponentenkopplungsteil mit dem Vorrichtungskopplungsteil gekoppelt ist.

3. Medizinisches Visualisierungssystem nach einem der vorstehenden Ansprüche, wobei die Übertragungskomponente ein Vorrichtungskabel (104) umfasst, das sich von einem ersten Kabelende (104A) zu einem zweiten Kabelende (104B) erstreckt, wobei das Komponentenkopplungsteil an dem zweiten Kabelende bereitgestellt ist.

4. Medizinisches Visualisierungssystem nach Anspruch 3, wobei das Vorrichtungskabel einen versteiften Abschnitt (116) umfasst, der sich von dem zweiten Kabelende in Richtung des ersten Kabelendes erstreckt, und optional, wobei der versteifte Abschnitt eine Länge von zwischen 20-150 mm von dem zweiten Kabelende aufweist.

5. Medizinisches Visualisierungssystem nach einem der Ansprüche 3-4, das eine Kabelrolle (400) umfasst, die so angepasst ist, dass sie mindestens einen Abschnitt des Vorrichtungskabels aufrollt.

6. Medizinisches Visualisierungssystem nach Anspruch 5, wobei die Kabelrolle ein Kabelrollengehäuse (406) umfasst, das den Abschnitt des Vorrichtungskabels umschließt, wenn es aufgerollt wird, und optional wobei die Kabelrolle einen UV-Licht-Emitter (410) umfasst, der in dem Kabelrollengehäuse untergebracht ist.

7. Medizinisches Visualisierungssystem nach einem der vorstehenden Ansprüche, wobei das Komponentenkopplungsteil ein primäres Kopplungselement (118) aufweist, das länglich ist und sich entlang einer Kopplungsteilachse erstreckt, und wobei das Vorrichtungskopplungsteil ein sekundäres Kopplungselement (226) aufweist, das einen Hohlraum bildet, der so angepasst ist, dass er das primäre Kopplungselement aufnimmt.

8. Medizinisches Visualisierungssystem nach Anspruch 7, wobei das Vorrichtungskopplungsteil einen Drehverschluss (228) umfasst, der so angepasst ist, dass er durch Drehen des Griffs in ein vorstehendes Element des Komponentenkopplungsteils eingreift, wenn das primäre Kopplungselement vollständig durch das sekundäre Kopplungselement aufgenommen ist.

9. Medizinisches Visualisierungssystem nach einem der vorstehenden Ansprüche, weiter umfassend einen Stützwinkel (300), der ein distales Stützwinkelteil (302) und ein proximales Stützwinkelteil (304) aufweist, und wobei das distale Stützwinkelteil ein Kopplungsteilrückhalteelement (306) einschließt, das so angepasst ist, dass es das Komponentenkopplungsteil der Übertragungskomponente zurückhält.

10. Medizinisches Visualisierungssystem nach Anspruch 9, wobei das proximale Stützwinkelteil so angepasst ist, dass es an das Gehäuse der Videoverarbeitungseinrichtung gekoppelt wird, d. h. so, dass der Stützwinkel der Drehung des Gehäuses um eine erste Achse folgt und der Drehung des Gehäuses um eine zweite Achse rechtwinklig zur ersten Achse nicht folgt.

11. Medizinisches Visualisierungssystem nach einem der Ansprüche 9-10, wobei das Kopplungsteilrückhalteelement so angepasst ist, dass es das Komponentenkopplungsteil durch lineare Bewegung des Komponentenkopplungsteils entlang einer dritten Achse aufnimmt, und/oder wobei das Komponentenkopplungsteil so angepasst ist, dass es in das Kopplungsteilrückhalteelement durch lineare Bewegung des Komponentenkopplungsteils entlang der dritten Achse eingreift.

12. Medizinisches Visualisierungssystem nach Anspruch 11, insofern abhängig von mindestens Anspruch 7, wobei die Kopplungsteilachse nicht parallel zu der dritten Achse ist, wie beispielsweise rechtwinkelig zu der dritten Achse, wenn das Komponentenkopplungsteil von dem Kopplungsteilrückhalteelement aufgenommen ist.

13. Medizinisches Visualisierungssystem nach einem der Ansprüche 9-12, insofern abhängig von mindestens Anspruch 5, wobei der Stützwinkel die Kabelrolle umfasst, wobei die Kabelrolle von einem Aktuator (402) angetrieben wird, und wobei der Aktuator aktiviert wird, um den Abschnitt des Vorrichtungskabels basierend auf einer ersten Steuerungseingabe aufzurollen, wobei die erste Steuerungseingabe dadurch ausgelöst wird, dass das Komponentenkopplungsteil von dem Kopplungsteilrückhalteelement aufgenommen wird, z. B. umfasst das Kopplungsteilrückhalteelement einen Schalter, der von der Aufnahme des Komponentenkopplungsteils abhängig ist, und wobei der Schalter die erste Steuerungseingabe auslöst.

## Revendications

1. Système de visualisation médicale (2) comprenant un appareil (20) de traitement vidéo, un endoscope (200) et un composant de transmission (100),
l'endoscope comprenant un module caméra (210) conçu pour générer des données d'image indiquant une vue provenant de l'endoscope, une poignée (202), et une partie (220) de couplage de dispositif,
l'appareil de traitement vidéo comprenant un boîtier (22) et un processeur (23) conçu pour recevoir les données d'image et/ou des données représentatives des données d'image et amener un dispositif d'affichage à afficher une représentation en direct des données d'image,
le composant de transmission comprenant un processeur (108) de composant conçu pour recevoir les données d'image provenant du module caméra et prétraiter les données d'image pour fournir les données représentatives des données d'image, le composant de transmission étant conçu pour transmettre les données représentatives des données d'image à l'appareil de traitement vidéo, le composant de transmission comprenant une partie (102) de couplage de composant conçue pour être couplée avec la partie de couplage de dispositif, dans lequel la partie de couplage de composant comprend le processeur de composant, dans lequel la partie de couplage de composant comprend un ou plusieurs mécanismes d'entrée physiques activables (114), et
dans lequel l'activation des un ou plusieurs mécanismes d'entrée physiques activables amène le composant de transmission à transmettre un ou plusieurs signaux de mécanisme d'entrée à l'appareil de traitement vidéo, indiquant l'activation des un ou plusieurs mécanismes d'entrée physiques activables, et
dans lequel la poignée de l'endoscope comprend un ou plusieurs éléments de communication (224), **caractérisé en ce que** les éléments de communication sont conçus pour activer les un ou plusieurs mécanismes d'entrée physiques activables lorsqu'ils sont manipulés par un utilisateur.

2. Système de visualisation médicale selon la revendication 1, dans lequel un premier élément de communication parmi les un ou plusieurs éléments de communication s'étend d'un premier côté (224A) à un second côté (224B), dans lequel le premier côté est agencé à l'extérieur de la poignée et le second côté est agencé à l'intérieur de la poignée, et dans lequel le second côté est conçu pour entrer en contact avec un premier mécanisme d'entrée physique activable parmi les un ou plusieurs mécanismes d'entrée physiques activables lorsque la partie de couplage de composant est couplée avec la partie de couplage de dispositif.

3. Système de visualisation médicale selon l'une quelconque des revendications précédentes, dans lequel le composant de transmission comprend un câble (104) de dispositif s'étendant d'une première extrémité (104A) de câble à une seconde extrémité (104B) de câble, dans lequel la partie de couplage de composant est disposée au niveau de la seconde extrémité de câble.

4. Système de visualisation médicale selon la revendication 3, dans lequel le câble de dispositif comprend une portion renforcée (116) s'étendant de la seconde extrémité de câble vers la première extrémité de câble, et éventuellement, dans lequel la portion renforcée présente une longueur de 20-150 mm à partir de la seconde extrémité de câble.

5. Système de visualisation médicale selon l'une quelconque des revendications 3 à 4, comprenant un enrouleur (400) de câble conçu pour enrouler au moins une portion du câble de dispositif.

6. Système de visualisation médicale selon la revendication 5, dans lequel l'enrouleur de câble comprend un boîtier (406) d'enrouleur de câble enfermant la portion du câble de dispositif lorsqu'elle est enroulée, et éventuellement, dans lequel l'enrouleur de câble comprend un émetteur (410) de lumière UV logé dans le boîtier d'enrouleur de câble.

7. Système de visualisation médicale selon l'une quelconque des revendications précédentes, dans lequel la partie de couplage de composant présente un élément de couplage primaire (118) étant allongé et s'étendant le long d'un axe de partie de couplage, et dans lequel la partie de couplage de dispositif présente un élément de couplage secondaire (226) formant une cavité conçue pour recevoir l'élément de couplage primaire.

8. Système de visualisation médicale selon la revendication 7, dans lequel la partie de couplage de dispositif comprend un verrou rotatif (228) conçu pour venir en prise, par rotation de la poignée, avec un élément saillant de la partie de couplage de composant lorsque l'élément de couplage primaire est entièrement reçu par l'élément de couplage secondaire.

9. Système de visualisation médicale selon l'une quelconque des revendications précédentes, comprenant en outre une patte de support (300) présentant une partie distale (302) de patte de support et une partie proximale (304) de patte de support, et dans lequel la partie distale de patte de support inclut un organe (306) de retenue de partie de couplage conçu pour retenir la partie de couplage de composant du composant de transmission.

10. Système de visualisation médicale selon la revendication 9, dans lequel la partie proximale de patte de support est conçue pour être couplée au boîtier de l'appareil de traitement vidéo, par exemple de sorte que la patte de support suive une rotation du boîtier autour d'un premier axe et ne suive pas une rotation du boîtier autour d'un deuxième axe perpendiculaire au premier axe.

11. Système de visualisation médicale selon l'une quelconque des revendications 9 à 10, dans lequel l'organe de retenue de partie de couplage est conçu pour recevoir la partie de couplage de composant par mouvement linéaire de la partie de couplage de composant le long d'un troisième axe, et/ou dans lequel la partie de couplage de composant est conçue pour venir en prise avec l'organe de retenue de partie de couplage par mouvement linéaire de la partie de couplage de composant le long du troisième axe.

12. Système de visualisation médicale selon la revendication 11, rattachée au moins à la revendication 7, dans lequel l'axe de partie de couplage est non parallèle au troisième axe, tel que perpendiculaire au troisième axe, lorsque la partie de couplage de composant est reçue par l'organe de retenue de partie de couplage.

13. Système de visualisation médicale selon l'une quelconque des revendications 9 à 12, rattachée au moins à la revendication 5, dans lequel la patte de support comprend l'enrouleur de câble, l'enrouleur de câble étant entraîné par un actionneur (402), et
dans lequel l'actionneur est activé pour enrouler la portion du câble de dispositif sur la base d'une première entrée de commande, dans lequel la première entrée de commande est déclenchée par la réception de la partie de couplage de composant par l'organe de retenue de partie de couplage, par exemple l'organe de retenue de partie de couplage comprend un commutateur assujetti à la réception de la partie de couplage de composant et le commutateur déclenche la première entrée de commande.
